# EUROPÄISCHE PATENTANMELDUNG

(11) **EP 1 972 626 A2**
(43) Veröffentlichungstag der Anmeldung: **24.09.2008**
(21) Anmeldenummer: 08159580.3
(22) Anmeldetag: 24.12.2002
(51) Int. Cl.: C07D 451/10

(54) **Verfahren zur Herstellung von Scopinestern**

(30) Priorität: 12.01.2002 DE 10200943
(62) Teilanmeldung aus: 02796735.5
(71) Anmelder: Boehringer Ingelheim Pharma GmbH & Co. KG, 55216 Ingelheim am Rhein (DE)
(72) Erfinder: Brandenburg, Jörg, 65199 Wiesbaden (DE); Pfrengle, Waldemar, 88400 Biberach (DE); Rall, Werner, 88441 Mittelbiberach (DE)
(74) Vertreter: Hammann, Heinz

(57) **Zusammenfassung**

Die Erfindung betrifft ein neues Verfahren zur Herstellung von Scopinestern der allgemeinen Formel 1 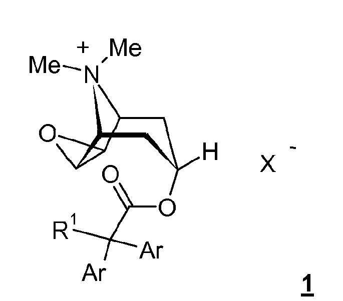 worin X - und die Reste R¹ und Ar die in den Ansprüchen und in der Beschreibung genannten Bedeutungen haben können.

## Beschreibung

Die Erfindung betrifft ein neues Verfahren zur Herstellung von Scopinestern der allgemeinen Formel 1 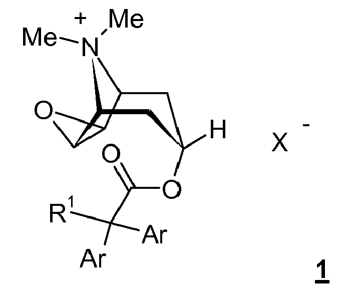 worin X - und die Reste R¹ und Ar die in den Ansprüchen und in der Beschreibung genannten Bedeutungen haben können.

### Hintergrund der Erfindung

Anticholinergika können bei einer Vielzahl von Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Zur Therapie dieser Erkrankungen werden beispielsweise durch die WO 92/16528 Anticholinergika vorgeschlagen, die ein Scopin-, Tropenol- oder auch Tropin-Grundgerüst aufweisen.

Die der WO 92/16528 zugrunde liegende Aufgabe zielt auf die Bereitstellung von anticholinerg wirksamen Verbindungen, die durch eine lang andauernde Wirksamkeit gekennzeichnet sind. Zur Lösung dieser Aufgabe werden durch die WO 92/16528 unter anderem Benzilsäureester des Scopins, Tropenols oder auch Tropins offenbart.

Zur Therapie chronischer Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei. Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Um als einmal täglich anwendbares Medikament zum Einsatz kommen zu können, sind an den zu applizierenden Wirkstoff besondere Anforderungen zu stellen. Zunächst sollte der nach Gabe des Arzneimittels erwünschte Wirkungseintritt relativ schnell erfolgen und im Idealfall über einen sich daran anschließenden längeren Zeitraum eine möglichst konstante Wirksamkeit aufweisen. Andererseits sollte die Wirkdauer des Arzneimittels einen Zeitraum von etwa einem Tag nicht wesentlich überschreiten. Im Idealfall zeigt ein Wirkstoff ein derart geartetes Wirkungsprofil, daß sich die Herstellung eines einmal täglich applizierbaren Arzneimittels, welches den Wirkstoff in therapeutisch sinnvollen Dosen enthält, gezielt steuern läßt.

Es wurde gefunden, daß die in der WO 92/16528 offenbarten Ester des Scopins, Tropenols oder auch Tropins diesen erhöhten Anforderungen nicht genügen. Sie sind aufgrund ihrer extrem langen Wirkungsdauer, die den vorstehend genannten Zeitraum von etwa einem Tag deutlich überschreiten, nicht als Einmalgabe pro Tag therapeutisch nutzbar.
Anders als die beispielsweise in der WO 92/16528 offenbarten Verbindungen wird die Herstellung von einmal täglich applizierbaren, anticholinerg wirksamen Arzneimitteln möglich, wenn Scopinester der Formel 1 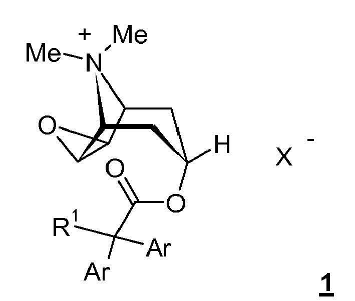 in denen X - und die Reste R¹ und Ar die nachstehend genannten Bedeutungen haben können, zum Einsatz gelangen.

Neben den in der WO 92/16528 offenbarten Syntheseverfahren zur Herstellung von Scopinestern werden beispielsweise auch in der EP 418 716 A1 Verfahren zur Herstellung von Estern des Scopins offenbart. Diese im Stand der Technik bekannten Verfahren sind auch zur Herstellung der Verbindungen der Formel 1 anwendbar. Allerdings handelt es sich bei diesen synthetischen Zugängen um teilweise komplexere, aus mehreren Synthesschritten bestehende Vorgehensweisen.

Aufgabe der vorliegenden Erfindung ist es, ein Syntheseverfahren bereitzustellen, welches einen einfacheren synthetischen Zugang zu den Verbindungen der allgemeinen Formel **1** ermöglicht.

### Detaillierte Beschreibung der Erfindung

Überraschenderweise wurde gefunden, daß Verbindungen der Formel 1 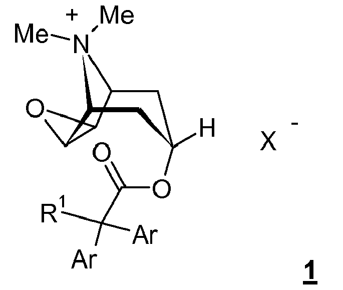 in denen X - und die Reste R¹ und Ar die nachstehend genannten Bedeutungen haben können, in einem einzigen Reaktionsschritt erhalten werden können, wenn als Ausgangsmaterial Verbindungen der Formel 2 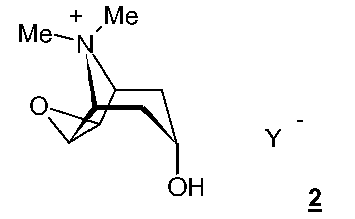 zum Einsatz gelangen.

Dementsprechend betrifft die vorliegende Erfindung ein Verfahren zur Herstellung von Verbindungen der Formel 1 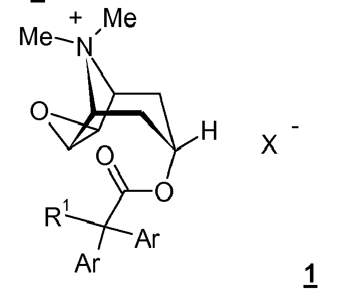 worin
- x -: Chlor, Brom, Iod, Methansulfonat oder Trifluormethansulfonat;
- R¹: Hydroxy, c₁-c₄-Alkyl, c₁-c₄-Alkoxy, CF₃ oder Fluor;
- Ar: ein Rest ausgewählt aus der Gruppe bestehend aus Phenyl, Naphthyl, Thienyl und Furyl, der gegebenenfalls ein- oder zweifach substituiert sein kann durch einen oder zwei Reste ausgewählt aus der Gruppe bestehend aus C₁-C₄-Alkyl, C₁-C₄-Alkoxy, Hydroxy, Fluor, Chlor, Brom oder CF₃, bedeuten können,
dadurch gekennzeichnet, daß eine Verbindung der Formel 2 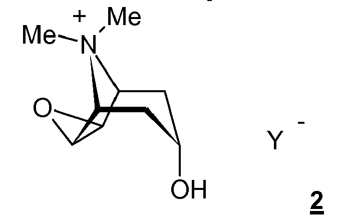 worin
- Y -: Chlor, Brom, Iod, Methansulfonat oder Trifluormethansulfonat bedeuten
kann in einem Schritt mit einer Verbindung der Formel 3 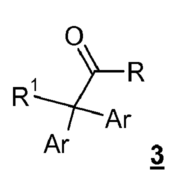
worin
- R: ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet und
die Reste R¹ und Ar eine der vorstehend genannten Bedeutungen haben können, umgesetzt wird.

Bevorzugt betrifft die vorliegende Erfindung ein Verfahren zur Herstellung von Verbindungen der Formel 1 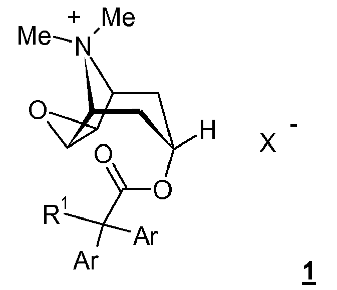 worin
- x -: Brom, Methansulfonat oder Trifluormethansulfonat;
- R¹: Hydroxy, Methyl, CF₃ oder Fluor;
- Ar: ein Rest ausgewählt aus der Gruppe bestehend aus Phenyl, Thienyl und Furyl, bedeuten können,
dadurch gekennzeichnet, daß eine Verbindung der Formel 2 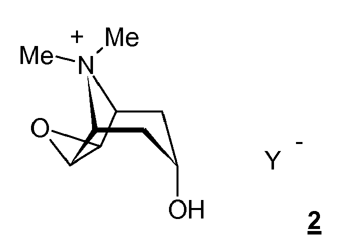 worin
- Y -: Brom, Methansulfonat oder Trifluormethansulfonat bedeuten kann
in einem Schritt mit einer Verbindung der Formel 3 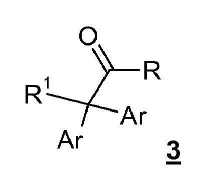 worin
- R: ein Rest ausgewählt aus der Gruppe bestehend aus Hydroxy, O-N-Succinimid, O-N-Phtalimid, Vinyloxy und 2-Allyloxy bedeutet und
die Reste R¹ und Ar eine der vorstehend genannten Bedeutungen haben können, umgesetzt wird.

Besonders bevorzugt betrifft die vorliegende Erfindung ein Verfahren zur Herstellung von Verbindungen der Formel 1 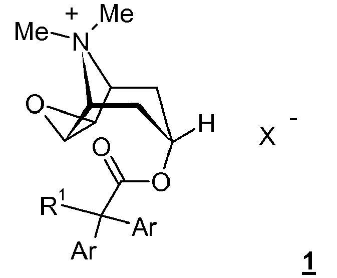 worin
- X -: Brom, Methansulfonat oder Trifluormethansulfonat;
- R¹: Hydroxy oder Methyl;
- Ar: Phenyl oder Thienyl bedeuten können,
dadurch gekennzeichnet, daß eine Verbindung der Formel 2 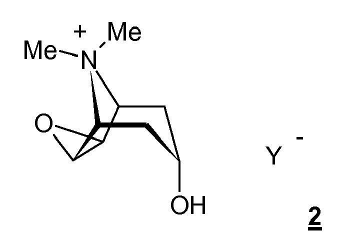 worin
- Y -: Brom, Methansulfonat oder Trifluormethansulfonat bedeuten kann
in einem Schritt mit einer Verbindung der Formel 3 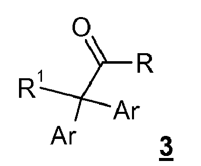 worin
- R: ein Rest ausgewählt aus der Gruppe bestehend aus Hydroxy, O-N-Succinimid, O-N-Phtalimid, Vinyloxy und 2-Allyloxy, bevorzugt Vinyloxy und 2-Allyloxy, bedeutet und
die Reste R¹ und Ar eine der vorstehend genannten Bedeutungen haben können, umgesetzt wird.

Zur Durchführung des erfindungsgemäßen Verfahrens kann wie nachfolgend beschrieben vorgegangen werden.
In einem ersten Schritt wird die Verbindung der Formel 3 in einem geeigneten organischen Lösmittel, vorzugsweise in einem polaren organischen Lösemittel, besonders bevorzugt in einem Lösemittel ausgewählt aus der Gruppe bestehend aus Acetonitril, Nitromethan, Formamid, Dimethylformamid, N-Methylpyrrolidinon, Dimethylsulfoxid und Dimethylacetamid aufgenommen, wobei von vorstehend genannten Lösemitteln Dimethylformamid, N-Methylpyrrolidinon und Dimethylacetamid besonders bevorzugt sind. Erfindungsgemäß von besonderer Bedeutung sind Dimethylformamid und N-Methylpyrrolidinon, wobei letzteres besonders bevorzugt ist.

Pro Mol engesetzte Verbindung der Formel 3 gelangen vorzugsweise zwischen 0,5 und 2 L, besonders bevorzugt zwischen 0,75 und 1,5 L des genannten Lösemittels zum Einsatz.

Je nach Wahl der Verbindung der Formel 3 kann es gegebenenfalls sinnvoll sein, diese vor Umsetzung mit der Verbindung der Formel 2 zu aktivieren. Werden als Verbindung der Formel 3 solche Derivate eingesetzt, in denen R =OH bedeutet, ist beispielsweise der Einsatz entsprechender Aktivierungsreagenzien wie Carbonyldiimidazol, Carbonyldi-1,2,4-triazol, Dicyclohexylcarbodiimid oder Ethyldimethylaminopropylcarbodiimid erfindungsgemäß bevorzugt, wobei in diesem Zusammenhang die Verwendung von Carbonyldiimidazol besonders bevorzugt ist. Pro Mol eingesetzte Verbindung **3** mit R=Hydroxy werden zwischen 1 - 2 Mol des Kopplungsreagenzes verwendet. Bevorzugt gelangen 1 - 1,5 Mol des Kopplungsreagenzes zum Einsatz. Gelangen vorstehend genannte Kopplungsreagentien, wie im Falle von R= Hydroxy bevorzugt, zum Einsatz wird die dann erhaltene Reaktionsmischung vorzugsweise in einem Temperaturbereich von 15-35°C, vorzugsweise bwi 20-25°C über einen Zeitraum von 1-8 Stunden, vorzugsweise 3-7 Stunden gerührt, bevor wie nachfolgend beschrieben weiter umgesetzt wird.

Die Reaktionsmischung von **3** in vorstehend genanntem Lösemittel, gegebenenfalls nach Zusatz eines der vorstehend genannten Kopplungsreagenzien im Falle von R=Hydroxy, wird sodann auf eine Temperatur von kleiner 30°C, vorzugsweise auf eine Temperatur zwischen -20°C und 20°C, besonders bevorzugt auf eine Temperatur zwischen -10°C und 5°C eingestellt und mit der Verbindung der Formel **2** versetzt. Bezogen auf ursprünglich eingesetzte Verbindung **3** können stöchiometrische Mengen an Verbindung der Formel **2** zugegeben werden. Erfindungsgemäß bevorzugt liegt **3** allerdings im Vergleich zu **2** im Überschuß vor. Pro Mol eingesetzte Verbindung **3** gelangen erfindungsgemäß zwischen 0,5 und **1** Mol, vorzugsweise zwischen 0,7 und 0,95 Mol, besonders bevorzugt zwischen 0,75 und 0,9 Mol **2** zur Anwendung.

Vorstehend genannte Reaktionsmischung wird anschließend mit einer in einem der vorstehend genannten Lösemittel gelösten geeigneten Base versetzt. Hierbei können organische oder anorganische Basen zum Einsatz gelangen. Als organische Basen werden bevorzugt Alkaliimidazolide verwendet, welche besiepielsweise in situ aus den Alkalimetallen und Imidazol oder den Alkalimetallhydriden und Imidazol generiert werden können. Als Akaliimidazolide kommen bevorzugt Imidazolide des Lithiums, Natriums oder Kaliums in Betracht, wobei Natrium- oder Lithiumimidazolid erfindungsgemäß bevorzugt sind. Besonders bevorzugt gelangt Lithiumimidazolid zum Einsatz. Als anorganische Base kommen vorzugsweise Hydride des Lithiums, Natriums oder Kaliums in Betracht. Besonders bevorzugt wird als anorganische Base Natriumhydrid eingesetzt. Von allen vorstehend genannten Basen gelangt besonders bevorzugt Lithiumimidazolid zur Anwendung.

Sollen Verbindungen der Formel **1** erhalten werden, in denen R¹ für Hydroxy steht steht, kann anstelle der vorstehend genannten basenkatalysierten Umsetzung auch eine Umesterung unter milderen Reaktionsbedingungen vorteilhaft erscheinen. Hierbei können vorteilhaft Zeolithe als Katalysatoren genutzt werden.

Wird die Umsetzung mit einer der vorstehend genannten Basen durchgeführt, werden pro Mol eingesetzte Verbindung **2** wenigstens stöchiometrische Mengen an Base eingesetzt. Bevorzugt werden pro Mol eingesetzte Verbindung **2** 1 bis 1,5 Mol, vorzugsweise 1,1 bis 1,3 Mol Base verwendet. Wird die Base in Form einer Lösung zugegeben, was insbesondere im Falle der erfindungsgemäß bevorzugten und zuvor in situ generierten Base Lithiumimidazolid der Fall ist, gelangt hierfür vorzugsweise dasjenige Lösemittel zur Anwendung, welches bereits zur Durchführung der vorstehend genannten Schritte Verwendung findet. Pro Mol engesetzte Base gelangen vorzugsweise zwischen 0,3 und 1,3 L, besonders bevorzugt zwischen 0,5 und 1 L des genannten Lösemittels zum Einsatz. Nach beendeter Basenzugabe wird in einem Temperaturbereich von 15-35°C, vorzugsweise bwi 20-25°C über einen Zeitraum von 4-48 Stunden, vorzugsweise 8-36 Stunden gerührt.

Zu der so entstandenen Suspension wird bei konstanter Temperatur eine Säure H-X gegeben. Die Wahl der Säure bestimmt sich dabei nach dem Anion X- im gewünschte Endprodukt der allgemeinen Formel **1.** Insofern im Rahmen der vorliegenden Erfindung bevorzugt solche Verbindungen der allgemeinen Formel **1** synthetisiert werden, in denen X- für Bromid steht, wird die nachfolgende Vorgehensweise für die Herstellung der erfindungsgemäß bevorzugten Bromidhaltigen Endprodukte der Formel **1** beschrieben. Für den Fachmann ist ersichtlich, daß eine entsprechende Vorgehensweise durch Wahl des geeigneten Reagenzes H-X in analoger Art und Weise auch zur Herstellung solcher Verbindungen Verwendung finden kann, in denen X- nicht Bromid bedeutet.

Zur Herstellung von Verbindungen der Formel **1** mit X- = Bromid werden bezogen auf eingesetzte Verbindung der Formel **3** vorzugsweise 2 bis 4 Mol, bevorzugt 2 bis 3 Mol, besonders bevorzugt 2,2 bis 2,6 Mol Bromwasserstoff bei konstanter Temperatur gegeben. Der verwendete Bromwasserstoff kann hierbei entweder in gasförmiger Form oder in Form, vorzugsweise gesättigter, Lösungen zugegeben werden. Erfindungsgemäß bevorzugt erfolgt die Zugabe an Bromwasserstoff in Eisessig gelöster Form. Besonders bevorzugt gelangt hierbei eine 33%-ige Bromwasserstoff-Lösung in Eisessig zum Einsatz. Nach beendeter Zugabe wird bei konstanter Temperatur, gegebenfalls auch unter Eiskühlung nachgerührt (zwischen 0,5 und 6 Stunden)

Abschließend wird die erhaltene Lösung mit einem unpolareren organischen Lösemittel, vorzugsweise mit einem Lösmittel ausgewählt aus der Gruppe bestehend aus Aceton, Toluol, n-Buthylacetat, Dichlormethan, Diethylether, Tetrahydrofuran und Dioxan, besonders bevorzugt Toluol oder Aceton versetzt.

Nach guter Durchmischung wird das auskristallisierte Produkt abgetrennt und mit dem vorstehend genannten unpolaren Lösemittel gewaschen. Zur Abtrennung wasserlöslicher Verunreinigungen kann das Rohprodukt mit wässerigen Bromidlösungen z.B. Natrium oder Kaliumbromidlösung behandelt werden.

Eine weitergehende Reinigung der so erhaltenen Verbindungen der Formel 1 kann, sofern erforderlich, durch Chromatographie über Kieselgel oder mittels Umkristallisation aus geeigneten Lösemitteln wie z.B. niederen Alkoholen, wie beispielsweise Isopropanol erfolgen.

Durch die Verwendung der im Stand der Technik bekannten Verbindungen der Formel 2 als Ausgangsmaterialien zur Synthese der Strukturen der Formel 1 gelingt ein Zugang zu diesen anticholinerg wirksamen Strukturen in nur einem Reaktionsschritt.
Dementsprechend betrifft ein weiterer Aspekt der vorliegenden Erfindung die Verwendung von Verbindungen der Formel 2 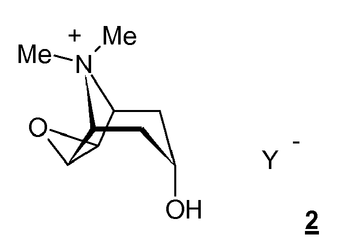 worin
- Y -: Chlor, Brom, Iod, Methansulfonat oder Trifluormethansulfonat bedeuten
als Ausgangsmaterial zur Herstellung von Verbindungen der Formel 1 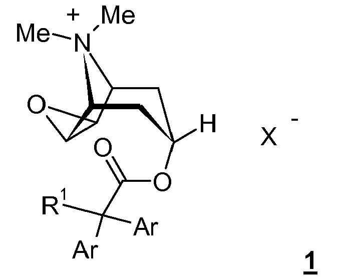 worin
- x -: Chlor, Brom, Iod, Methansulfonat oder Trifluormethansulfonat;
- R¹: Hydroxy, c₁-c₄-Alkyl, c₁-c₄-Alkoxy, CF₃ oder Fluor;
- Ar: ein Rest ausgewählt aus der Gruppe bestehend aus Phenyl, Naphthyl, Thienyl und Furyl, der gegebenenfalls ein- oder zweifach substituiert sein kann durch einen oder zwei Reste ausgewählt aus der Gruppe bestehend aus C₁-C₄-Alkyl, C₁-C₄-Alkoxy, Hydroxy, Fluor, Chlor, Brom oder CF₃, bedeuten können.

Bevorzugt betrifft die vorliegende Erfindung die Verwendung von Verbindungen der Formel 2 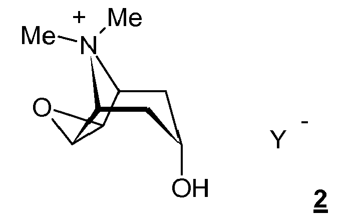 worin
- Y -: Brom, Methansulfonat oder Trifluormethansulfonat bedeuten
als Ausgangsmaterial zur Herstellung von Verbindungen der Formel 1 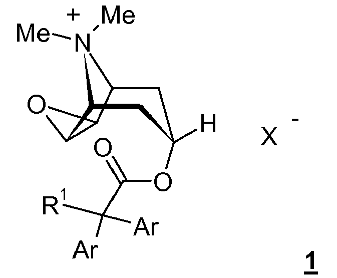 worin
- X -: Brom, Methansulfonat oder Trifluormethansulfonat;
- R¹: Hydroxy, Methyl, CF₃ oder Fluor;
- Ar: ein Rest ausgewählt aus der Gruppe bestehend aus Phenyl, Thienyl und Furyl, bedeuten können.

Besonders bevorzugt betrifft die vorliegende die Verwendung von Verbindungen der Formel 2 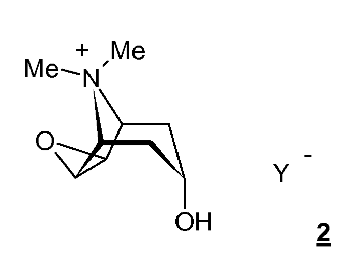 worin
- Y -: Brom, Methansulfonat oder Trifluormethansulfonat bedeuten
als Ausgangsmaterial zur Herstellung von Verbindungen der Formel 1 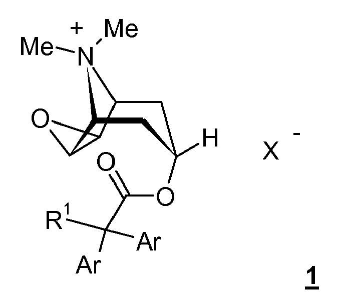 worin
- x⁻: Brom, Methansulfonat oder Trifluormethansulfonat;
- R¹: Hydroxy oder Methyl;
- Ar: Phenyl oder Thienyl bedeuten können.

Die nachfolgenden Beispiele dienen der Illustration exemplarisch durchgeführter Syntheseverfahren. Sie sind lediglich als mögliche, exemplarisch dargestellte Vorgehensweisen zu verstehen, ohne die Erfindung auf deren Inhalt zu beschränken.

### Beispiel 1: 2.2-Diphenylpropionsäurescopinester-Methobromid :

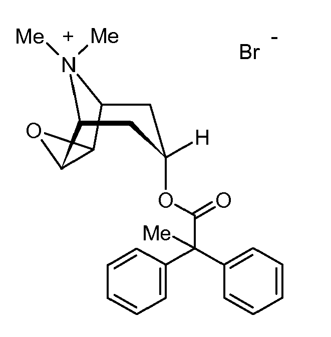

Zu einer Lösung von 2,2-Diphenylpropionsäure (1629 g, 7,2 mol ) in N-Methylpyrrolidinon ( 9 L ) wird Carbonyldiimidazol (1206 g, 7,44 mol ) portionsweise zugegeben und anschließend für 5 Stunden bei Raumtemperatur (etwa 23°C) gerührt. Das Reaktionsgemisch wird auf -3°C abgekühlt. Scopinmethobromid (1501g, 6,0 mol ) wird zum Reaktionsgemisch gegeben. Anschließend wird eine Lösung von Lithiumimidazolid (hergestellt aus Lithiumhydrid ( 59,6g; 7,12 mol ) sowie Imidazol (490.2 g, 7,2 mol ) in 5 L N-Methylpyrrolidinon zugetropft. Es wird bei Raumtemperatur für 17 Stunden gerührt. Zur entstandenen Suspension wird Bromwasserstoff-Lösung (33% in Eisessig; 2460 ml, 14,25 mol ) bei 18-28°C unter Kühlung zugegeben. Die Suspension wird im Eisbad nachgerührt und anschließend mit Toluol ( 14 L ) versetzt. Es wird filtriert und der erhaltene Filterkuchen zweimal mit je 5500 ml 30%iger Kaliumbromidlösung aufgeschlämmt und abgesaugt. Die so erhaltene Substanz wird im Trockenschrank bei 40°C getrocknet.
Ausbeute: 2359.3 g = 85.8 % d.Th.

Zur Reinigung wird das Rohprodukt (2100 g ) aus 35.7 L Isopropanol umkristallisiert. Ausbeute: 1562.2 g; farblose Blättchen;

In analoger Art und Weise kann in einem Syntheseschritt erhalten werden:

### Beispiel 2: (1α,2β,4β,5α,7β)-7-[(Hydroxydi-2-thienylacetyl)oxy]-9,9-dimethyl-3-oxa-9-azoniatricyclo[3.3.1.0^{2,4}]nonane-bromid

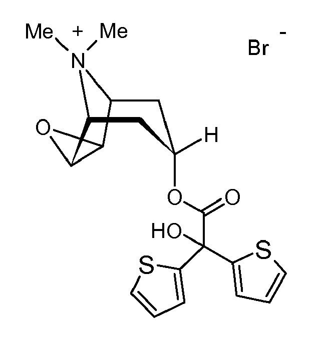

## Patentansprüche

1. Verfahren zur Herstellung von Verbindungen der Formel **1** 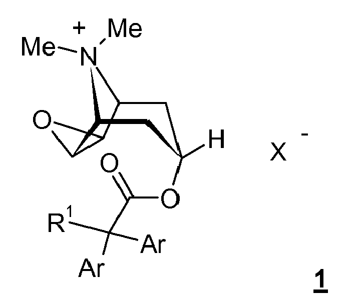 worin
X - Chlor, Brom, Iod, Methansulfonat oder Trifluormethansulfonat;
R¹ Hydroxy, C₁-C₄-Alkyl, C₁-C₄-Alkoxy, CF₃ oder Fluor;
Ar ein Rest ausgewählt aus der Gruppe bestehend aus Phenyl, Naphthyl, Thienyl und Furyl, der gegebenenfalls ein- oder zweifach substituiert sein kann durch einen oder zwei Reste ausgewählt aus der Gruppe bestehend aus c₁-c₄-Alkyl, C₁-C₄-Alkoxy, Hydroxy, Fluor, Chlor, Brom oder CF₃, bedeuten können,
**dadurch gekennzeichnet, daß** eine Verbindung der Formel 2 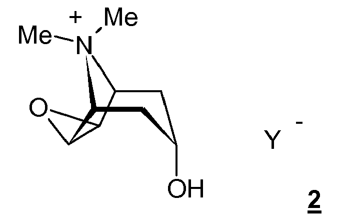 worin
Y - Chlor, Brom, Iod, Methansulfonat oder Trifluormethansulfonat bedeuten kann,
in einem Schritt mit einer Verbindung der Formel 3 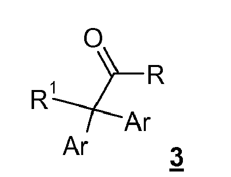 worin
R ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet und
die Reste R¹ und Ar eine der vorstehend genannten Bedeutungen haben können, umgesetzt wird.

2. Verfahren nach Anspruch 1, zur Herstellung von Verbindungen der Formel **1** worin
X - Brom, Methansulfonat oder Trifluormethansulfonat;
R¹ Hydroxy, Methyl, CF₃ oder Fluor;
Ar ein Rest ausgewählt aus der Gruppe bestehend aus Phenyl, Thienyl und Furyl, bedeuten können,
**dadurch gekennzeichnet, daß** eine Verbindung der Formel **2** worin
Y - Brom, Methansulfonat oder Trifluormethansulfonat bedeuten kann,
in einem Schritt mit einer Verbindung der Formel **3**, worin
R ein Rest ausgewählt aus der Gruppe bestehend aus Hydroxy, O-N-Succinimid, O-N-Phtalimid, Vinyloxy und 2-Allyloxy bedeutet und
die Reste R¹ und Ar eine der vorstehend genannten Bedeutungen haben können, umgesetzt wird.

3. Verfahren nach Anspruch 1 oder 2 zur Herstellung einer Verbindung der Formel **1** , worin
X - Brom, Methansulfonat oder Trifluormethansulfonat;
R¹ Hydroxy oder Methyl;
Ar Phenyl oder Thienyl bedeuten können,
**dadurch gekennzeichnet, daß** eine Verbindung der Formel **2**, worin
Y - Brom, Methansulfonat oder Trifluormethansulfonat bedeuten kann,
in einem Schritt mit einer Verbindung der Formel **3**, worin
R ein Rest ausgewählt aus der Gruppe bestehend aus Hydroxy, O-N-Succinimid, O-N-Phtalimid, Vinyloxy und 2-Allyloxy, bevorzugt Vinyloxy und 2-Allyloxy, bedeutet und
die Reste R¹ und Ar eine der vorstehend genannten Bedeutungen haben können, umgesetzt wird.

4. Verfahren nach einem der Ansprüche 1 bis 3, **dadurch gekennzeichnet, daß** die Umsetzung in einem organischen Lösmittel ausgewählt aus der Gruppe bestehend aus Acetonitril, Nitromethan, Formamid, Dimethylformamid, N-Methylpyrrolidinon, Dimethylsulfoxid und Dimethylacetamid durchgeführt wird.

5. Verfahren nach einem der Ansprüche 1 bis 4, **dadurch gekennzeichnet, daß** im Falle der Verwendung einer Verbindung der Formel **3**, in der R =OH bedeutet, Aktivierungsreagenzien ausgewählt aus der Gruppe Carbonyldiimidazol, Carbonyldi-1,2,4-triazol, Dicyclohexylcarbodiimid und Ethyldimethylaminopropylcarbodiimid zur Anwendung gelangen.

6. Verfahren nach einem der Ansprüche 1 bis 5, **dadurch gekennzeichnet, daß** die Umsetzung bei einer Temperatur von kleiner 30°C, vorzugsweise auf eine Temperatur zwischen -20°C und 20°C durchgeführt wird.

7. Verfahren nach einem der Ansprüche 1 bis 6, **dadurch gekennzeichnet, daß** die Umsetzung in Gegenwart einer organischen oder anorganischen Base durchgeführt wird.

8. Verfahren nach einem der Ansprüche 1 bis 6, **dadurch gekennzeichnet, daß** in dem Fall, in dem in den Verbindungen der Formel **1** R¹ für Hydroxy steht, die Umsetzung in Gegenwart von Zeolithe als Katalysator durchgeführt wird.

9. Verwendung einer Verbindung der Formel **2** 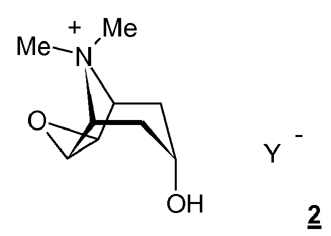 worin
Y - Chlor, Brom, Iod, Methansulfonat oder Trifluormethansulfonat bedeutet,
als Ausgangsmaterial zur Herstellung von Verbindungen der Formel **1** 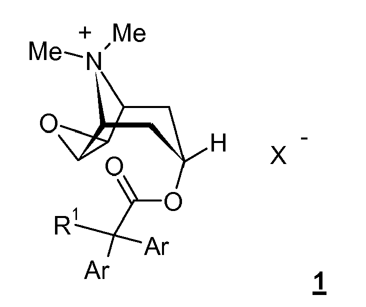 worin
x - Chlor, Brom, Iod, Methansulfonat oder Trifluormethansulfonat;
R¹ Hydroxy, C₁-C₄-Alkyl, C₁-C₄-Alkoxy, CF₃ oder Fluor;
Ar ein Rest ausgewählt aus der Gruppe bestehend aus Phenyl, Naphthyl, Thienyl und Furyl, der gegebenenfalls ein- oder zweifach substituiert sein kann durch einen oder zwei Reste ausgewählt aus der Gruppe bestehend aus C₁-C₄-Alkyl, C₁-C₄-Alkoxy, Hydroxy, Fluor, Chlor, Brom oder CF₃, bedeuten können.
